# EUROPEAN PATENT APPLICATION

(11) **EP 3 428 261 A1**
(43) Date of publication of application: **16.01.2019**
(21) Application number: 16893527.8
(22) Date of filing: 11.03.2016
(51) Int. Cl.: C12M 1/00

(54) **IMAGE PROCESSING DEVICE, OBSERVATION DEVICE, AND PROGRAM**

(71) Applicant: Nikon Corporation, Tokyo 108-6290 (JP)
(72) Inventor: KII Hiroaki, Tokyo 108-6290 (JP); KIYOTA Yasujiro, Tokyo 108-6290 (JP); UOZUMI Takayuki, Tokyo 108-6290 (JP); KAWASHIMA Kazuhiro, Tokyo 140-0015 (JP); YAMAZAKI Yoichi, Tokyo 140-0015 (JP)
(74) Representative: Hoffmann Eitle
(86) International application number: PCT/JP2016/057768
(87) International publication number: WO 2017/154200

(57) **Abstract**

An image processing device includes: an associating unit configured to associate information on an identification element used to identify a cell in imaged cell information with information on a non-identification element serving as an element of the cell which is not the identification element on the basis of the information on the identification element and information of the non-identification element; and a status determination unit configured to determine a cell status on the basis of the information on the identification element and the information on the non-identification element associated together by the linking unit.

## Description

### [Technical Field]

The present invention relates to an image processing device, an observation device, and a program.

### [Background Art]

Generally, a technique for evaluating a cell culture status is a fundamental technique in a wide range of fields including advanced medical fields such as regenerative medicine and screening of pharmaceuticals. For example, in the field of regenerative medicine, there are processes of growing and differentiating cells in vitro. Moreover, in the above-described process, it is necessary to accurately evaluate a cell culture status such as success or failure of differentiation, canceration of cells, and presence or absence of infection. For example, a method for determining a cell culture status by performing image processing on an image having a cell captured therein has been disclosed (refer to Patent Document 1).

### [Citation List]

### [Patent Literature]

[Patent Document 1]
Specification of United States Patent Application, Publication No. 2011/0206643

### [Summary of Invention]

### [Technical Problem]

With regard to a cell culture status, it is sometimes desired to evaluate from which cell, among a plurality of cells, a structure of a cell such as a neurite is derived. However, the origination cell of a cell structure cannot be determined from a plurality of cells by the above-described conventional technique. Thus, there is a problem that labor is required for the evaluation. In other words, the above-described conventional techniques have a problem in that evaluation of the culture status of the cells is laborious.

The present invention was made in view of the above-described problems and an objective of the present invention is to provide an image processing device, an observation device, and a program capable of reducing the labor of evaluating a cell culture status.

### [Solution to Problem]

In order to solve the above-described problems, an aspect of the present invention is an image processing device including: an associating unit configured to associate information on an identification element used to identify a cell in imaged cell information with information on a non-identification element which is an element of the cell which is not the identification element on the basis of the information on the identification element and information of the non-identification element; and a status determination unit configured to determine a status of the cell on the basis of the information on the identification element and the information on the non-identification element associated together by the linking unit.

Also, in order to solve the above-described problems, an observation device includes an imaging unit configured to output an image having a cell captured therein; and the above-described image processing device.

In order to solve the above-described problems, an aspect of the present invention is a program which causes a computer to execute: an association step of associating information on an identification element used to identify a cell in a captured cell image with information on a non-identification element serving as an element of the cell which is not the identification element on the basis of the identification element and the information on the non-identification element; and a status determination step of determining a status of the cell on the basis of the information on the identification element and the information on the non-identification element associated with each other in the association step.

### [Advantageous Effects of Invention]

According to the present invention, it is possible to reduce the labor of evaluating a cell culture status.

### [Brief Description of Drawings]

Fig. 1 is a schematic diagram showing an example of a constitution of an observation device according to an embodiment of the present invention.
Fig. 2 is a block diagram showing a functional constitution of an image processing device in the embodiment.
Fig. 3 is a diagram showing an example of an operation of the image processing device in the embodiment.
Fig. 4 is a diagram showing an example of an observation image captured by an imaging unit in the embodiment.
Fig. 5 is a diagram showing an example of a non-identification element included in the observation image in the embodiment.
Fig. 6 is a diagram showing an example of an image of a non-identification element with a first pattern extracted by a non-identification element extraction unit in the embodiment.
Fig. 7 is a diagram showing an example of an image of a non-identification element with a second pattern extracted by the non-identification element extraction unit in the embodiment.
Fig. 8 is a diagram showing an example of an image obtained by superimposing an image of a first pattern element and an image of a second pattern element extracted by the non-identification element extraction unit in the embodiment.
Fig. 9 is a diagram showing an example of an image mask generated by a mask generation unit in the embodiment.
Fig. 10 is a diagram showing an example of a linking result by a linking unit in the embodiment.
Fig. 11 is a diagram showing an example of a determination operation by a status determination unit in the embodiment.
Fig. 12 is a diagram showing an example of a determination result by a status determination unit in the embodiment.

### [Description of Embodiments]

### [Constitution of observation device 1]

A constitution of an observation device 1 according to an embodiment will be described below with reference to the drawings.

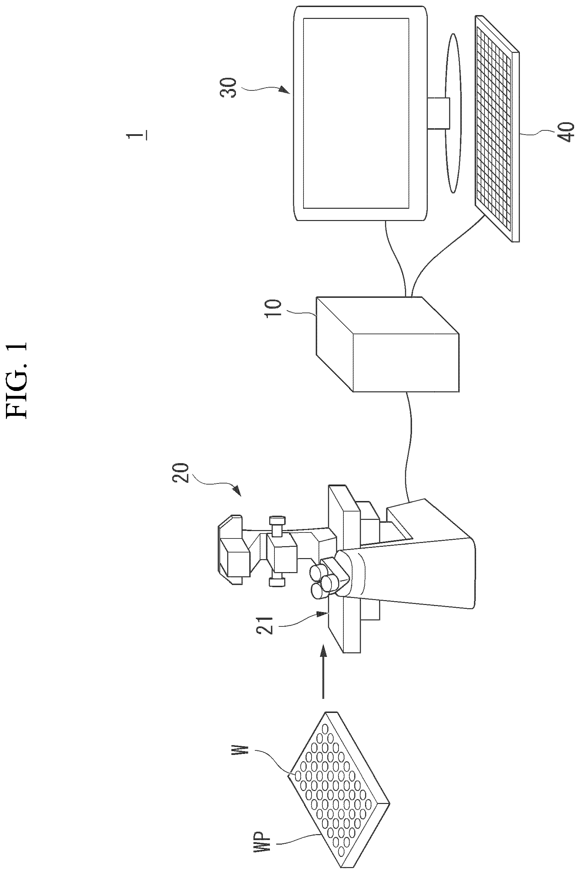

Fig. 1 is a schematic diagram showing an example of the constitution of the observation device 1 according to the embodiment of the present invention.

The observation device 1 performs image processing on an image obtained by capturing cells or the like. In the following description, an image obtained by capturing cells or the like is also simply referred to as a "cell image." The observation device 1 includes an image processing device 10, a microscope device 20, a display unit 30, and an operation detection unit 40.

The microscope device 20 is a biomicroscope and includes a motorized stage 21 and an imaging unit 22. The motorized stage 21 can arbitrarily move a position of an object to be imaged in a predetermined direction (for example, any direction in a two-dimensional horizontal plane). The imaging unit 22 includes an imaging element such as a charge-coupled device (CCD) or a complementary metal-oxide semiconductor (CMOS) and captures the object to be imaged on the motorized stage 21. It should be noted that the microscope device 20 may not include the motorized stage 21 and the stage may be a stage that does not move in a predetermined direction.

To be more specific, the microscope device 20 functions, for example, as a differential interference contrast microscope (DIC), a phase difference microscope, a fluorescence microscope, a confocal microscope, or a super-resolution microscope. The microscope device 20 captures an image of a culture container placed on the motorized stage 21. The culture container is, for example, a well plate WP. The microscope device 20 irradiates cells cultured in a plurality of wells W included in the well plate WP with light and captures an image of transmitted light transmitted through the cells as an image of the cells. Thus, it is possible to acquire an image such as a transmission DIC image, a phase difference image, a dark field image, or a bright field image of cells. In addition, an image of the fluorescence emitted from a biological substance when cells are irradiated with excitation light which excites a fluorescent substance is captured as an image of the cells. Furthermore, the microscope device 20 may capture an image of the fluorescence emitted from a coloring substance itself contained in a biological substance or an image of the fluorescence emitted when a substance with a chromophore binds to a biological substance as the above-described image of the cells. Thus, the observation device 1 can acquire a fluorescence image, a confocal image, or a super-resolution image. It should be noted that a method for acquiring an image of cells is not limited to an optical microscope.

The well plate WP has the plurality of wells W. In this example, the well plate WP has 96 wells W of 12×8. Cells are cultured in the wells W under specific experimental conditions. The specific experimental conditions include a temperature, a humidity, a culture period, an elapsed time after a stimulus is applied, a type and an intensity of stimulus to be applied, the presence or absence of a stimulus, induction of biological features, and the like. A stimulus is, for example, a physical stimulus such as an electric, acoustic, magnetic, or light stimulus, a chemical stimulus through administration of a substance or a medicine, or the like. Furthermore, biological features are features which indicate a stage of cell differentiation, morphology, the number of cells, and the like.

The display unit 30 includes a liquid crystal display or the like and displays a calculation result by the image processing device 10.

The operation detection unit 40 includes a mouse (not shown) and a keyboard and detects an operation performed for the image processing device 10.

### [Functional constitution of image processing device 10]

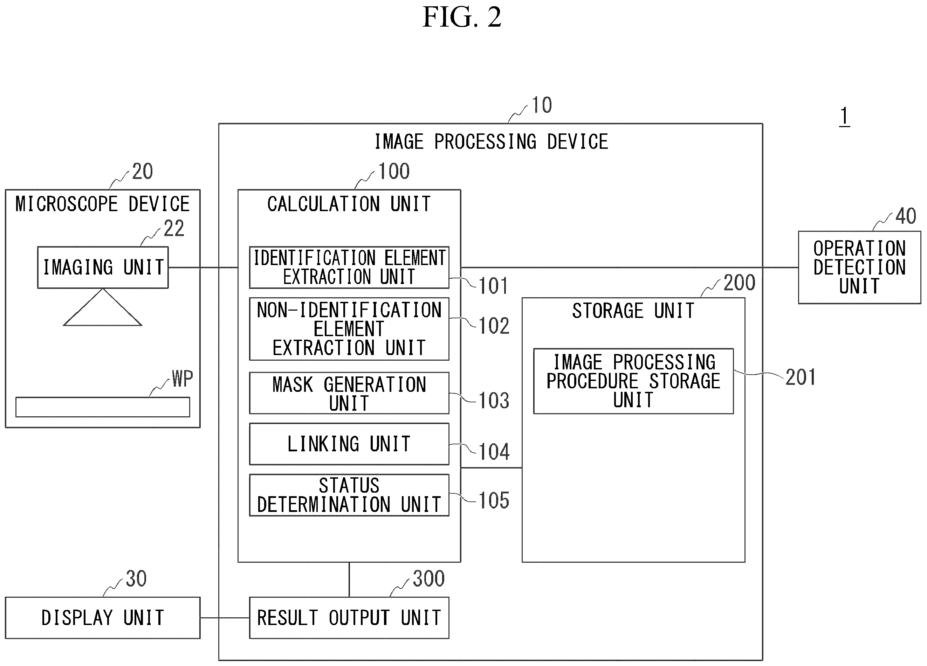

Fig. 2 is a block diagram showing a functional constitution of the image processing device 10 in the embodiment. The image processing device 10 includes a calculation unit 100 and a storage unit 200.

The storage unit 200 includes an image processing procedure storage unit 201.

The image processing procedure storage unit 201 stores a processing procedure for an image to be processed in the image processing device 10.

Also, the storage unit 200 stores a control program for the microscope device 20 in advance.

The calculation unit 100 includes a central processing unit (CPU) and drives each unit in the microscope device 20 in accordance with the control program stored in the storage unit 200. Since the detailed content of control for the microscope device 20 by the calculation unit 100 is known, a description thereof will be omitted.

Also, the calculation unit 100 includes an identification element extraction unit 101, a non-identification element extraction unit 102, a mask generation unit 103, a linking unit 104, and a status determination unit 105 as its functional units.

The identification element extraction unit 101 extracts, for example, an image which is information on an identification element from an image captured by the imaging unit 22. The identification element is an element, among elements that constitute a cell, used to identify cells one by one in an image in which a plurality of cells are captured. The identification element is used for identifying a cell (for example, labeling it in an image). The identification element is preferably distinguishable from other elements in a cell. To be specific, the identification element is a cell nucleus, a cell membrane, a cell body, an intracellular organelle, or the like. In the following example, a case in which the identification element is a "cell nucleus" will be described.

To be specific, the imaging unit 22 captures cells cultured in the wells W of the well plate WP. That is to say, an image captured by the imaging unit 22 includes an image of a cell nucleus. The identification element extraction unit 101 extracts an image of a cell nucleus from an image captured by the imaging unit 22 by performing image processing on an image captured by the imaging unit 22 by a known method such as pattern matching. Here, a cell nucleus may be stained by a marker such as 4',6-diamidino-2-phenylindole (DAPI) in some cases. In this case, the identification element extraction unit 101 extracts a portion stained by DAPI as an image of a cell nucleus in an image captured by the imaging unit 22. The identification element extraction unit 101 supplies the extracted image of the cell nucleus to each functional unit in the calculation unit 100.

The non-identification element extraction unit 102 extracts, for example, an image which is information on an element which is not the identification element among elements constituting a cell from an image captured by the imaging unit 22. Hereinafter, elements which are not the identification element are also referred to as "non-identification elements." Furthermore, in the following example in which an identification element is a "cell nucleus," elements which are not the identification element are also referred to as "non-nucleus elements." When a cell captured by the imaging unit 22 is a neuron, non-nucleus elements include a dendrite, an axon, and the like. The non-nucleus elements may be stained by a marker which is appropriate for each element in some cases. For example, an element expressed in a neuron may be stained by a marker such as nestin in some cases. In this case, the non-identification element extraction unit 102 extracts a portion stained by the nestin as an image of a non-nucleus element in an image captured by the imaging unit 22. The non-identification element extraction unit 102 supplies the extracted image of the non-nucleus element to each functional unit in the calculation unit 100.

The mask generation unit 103 generates an image mask for performing image processing on an image captured by the imaging unit 22. To be specific, the mask generation unit 103 generates an image mask corresponding to an image of the non-nucleus element on the basis of the image of the non-nucleus element extracted by the non-identification element extraction unit 102.

The linking unit 104 associates (or "links") an image of a cell nucleus extracted by the identification element extraction unit 101 with an image of the non-nucleus element on the basis of the image of the non-nucleus element extracted by the non-identification element extraction unit 102.

The status determination unit 105 determines a status of the cell on the basis of the image of the cell nucleus and the image of the non-nucleus element linked by the linking unit 104.

### [Operation of image processing device 10]

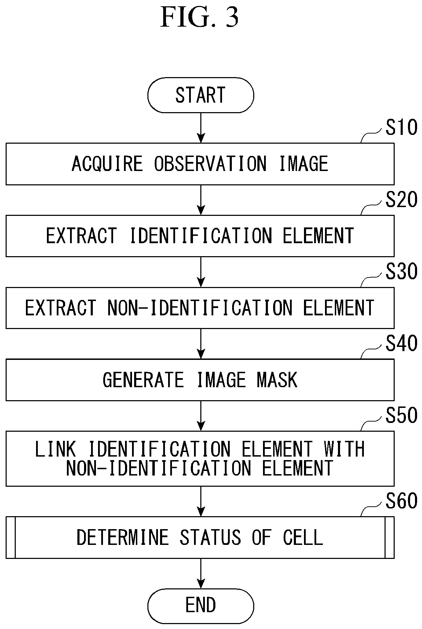

An example of an operation of the image processing device 10 will be described below with reference to Fig. 3.

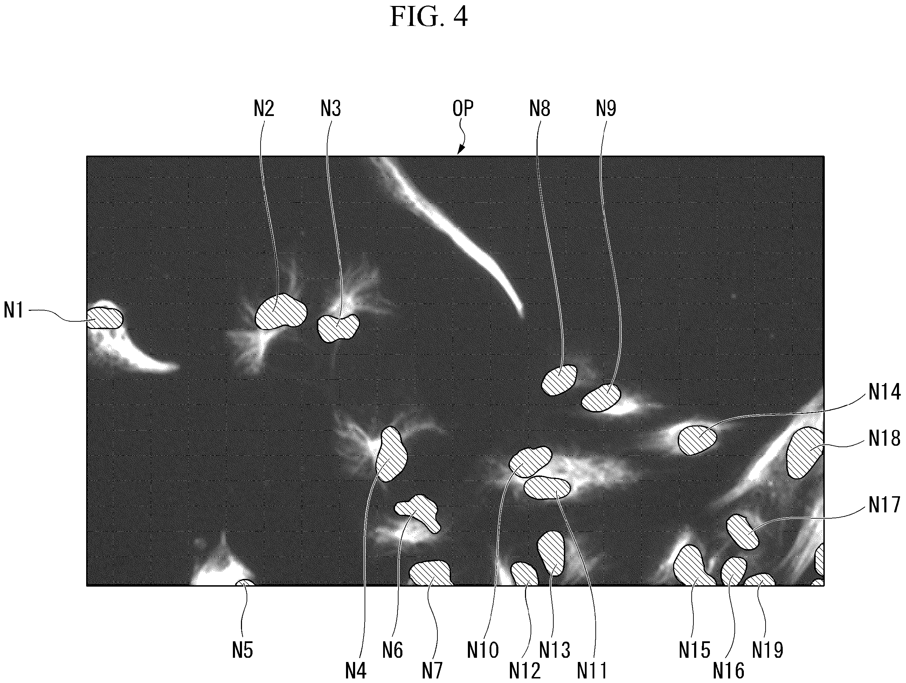

Fig. 3 is a diagram showing an example of an operation of the image processing device 10 in the embodiment. When the imaging unit 22 captures a cell in the well plate WP, the calculation unit 100 acquires an observation image OP captured by the imaging unit 22 (Step S10). Fig. 4 shows an example of the observation image OP.

Fig. 4 is a diagram showing the example of the observation image OP captured by the imaging unit 22 in the embodiment. The observation image OP includes an image of a cell whose cell nucleus is stained by DAPI.

The identification element extraction unit 101 extracts a portion stained by DAPI as an image of the cell nucleus from the observation image OP (Step S20). In a specific example in Fig. 4, the identification element extraction unit 101 extracts 19 images of the cell nucleus from a cell nucleus image N1 to a cell nucleus image N19.

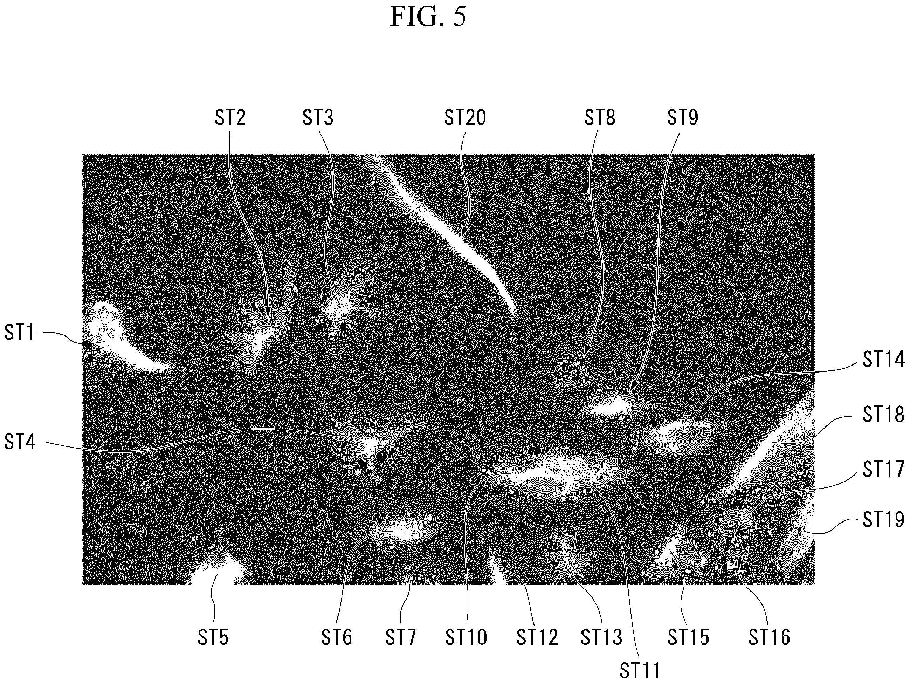

Referring again to Fig. 3, the non-identification element extraction unit 102 acquires the observation image OP captured by the imaging unit 22. The observation image OP includes a non-nucleus element in some cases. Fig. 5 shows an example of an image of the non-nucleus element included in the observation image OP.

Fig. 5 is a diagram showing an example of a non-nucleus element included in the observation image OP in the embodiment. In this example, 19 comparison elements from a non-nucleus element ST1 to a non-nucleus element 19 are included in the observation image OP. The non-identification element extraction unit 102 extracts a non-nucleus element from the observation image OP (Step S30). In the example shown in Fig. 5, the non-identification element extraction unit 102 extracts 19 non-nucleus elements, from the non-nucleus element ST1 to the non-nucleus element 19, from the observation image OP.

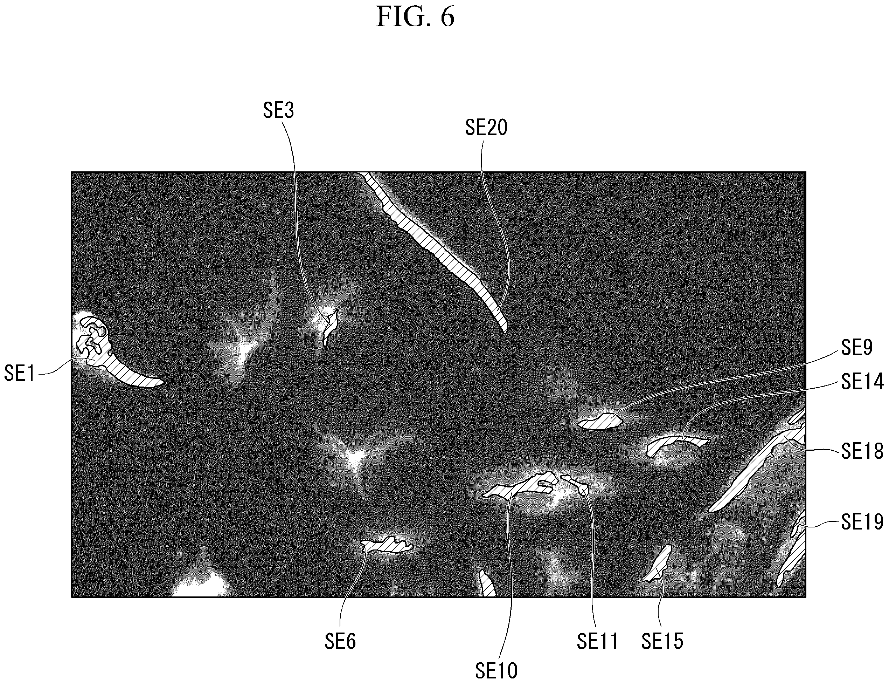

There are various kinds of non-nucleus elements. In this example, the non-nucleus elements include a non-nucleus element with a first pattern P1 and a non-nucleus element with a second pattern P2. An image of the non-nucleus element with the first pattern P1 includes a fibrous pattern appearing therein in some cases. Here, the fibrous pattern is a pattern whose aspect ratio is equal to or greater than a predetermined value, that is, an elongated pattern, and whose length in a longitudinal direction is equal to or greater than a predetermined value among patterns appearing in an image. Furthermore, in an image of the non-nucleus element with the second pattern P2, the brightness of an image of the non-nucleus element is greater than that of an image around the non-nucleus element in some cases. The non-identification element extraction unit 102 extracts the fibrous pattern as the non-nucleus element with the first pattern P1 when the observation image OP includes the fibrous pattern. Furthermore, when there is a relatively bright image region in the observation image OP, the non-identification element extraction unit 102 extracts this bright image region as the non-nucleus element with the second pattern P2. Fig. 6 shows an example of the image of the non-nucleus element with the first pattern P1. In the following description, the non-nucleus element with the first pattern P1 is also simply referred to as a "first pattern element."

Fig. 6 is a diagram showing an example of the image of the non-nucleus element with the first pattern P1 extracted by the non-identification element extraction unit 102 in the embodiment. In this example, the non-identification element extraction unit 102 extracts 11 first pattern elements from a first pattern element SE1 to a first pattern element SE20 shown in Fig. 6.

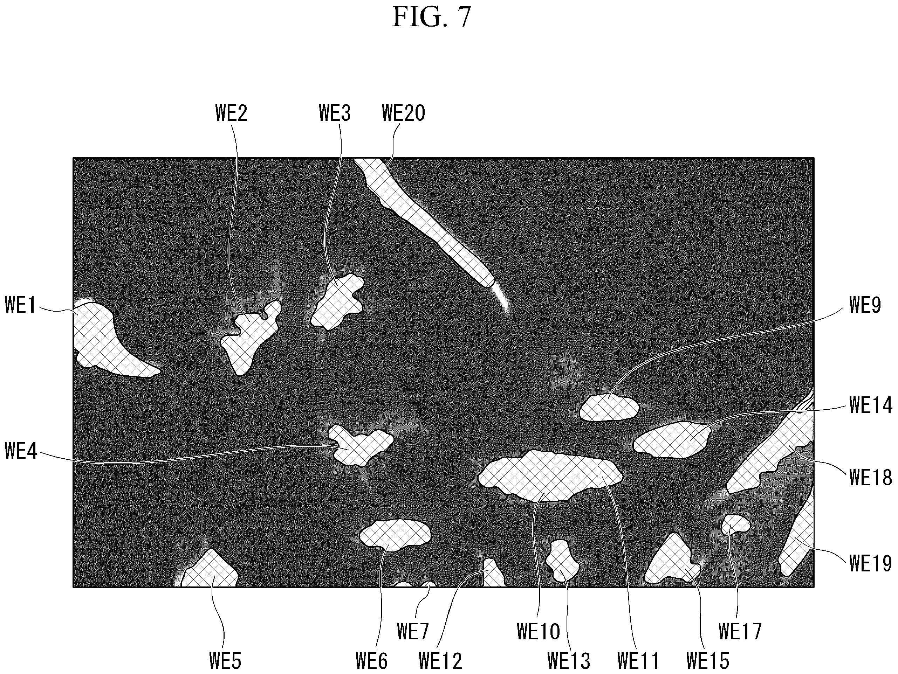

Also, Fig. 7 shows an example of the image of the non-nucleus element with the second pattern P2. In the following description, the non-nucleus element with the second pattern P2 is also simply referred to as a "second pattern element."

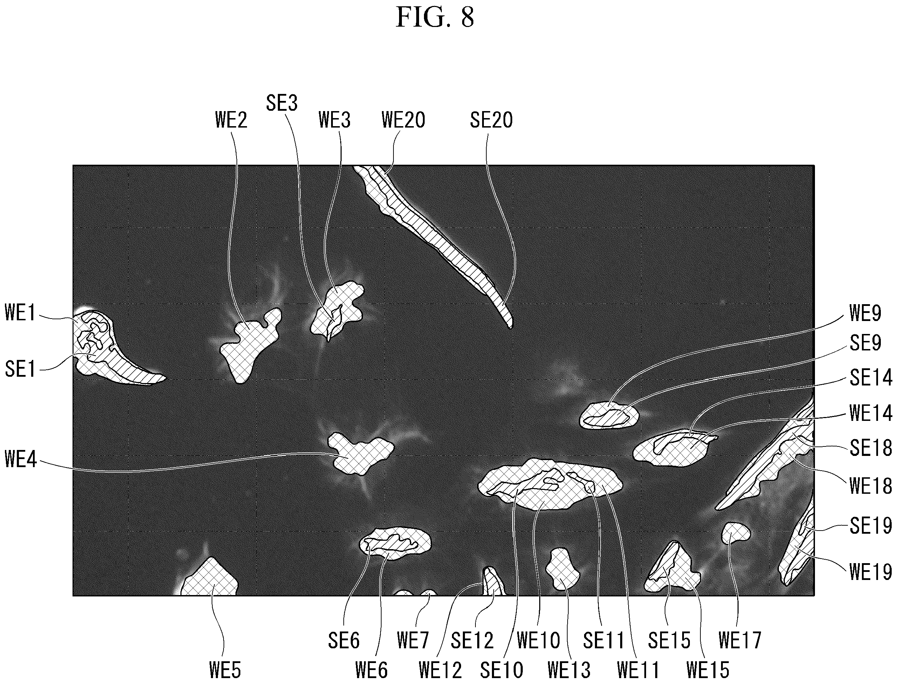

Fig. 7 is a diagram showing an example of the image of the non-nucleus element with the second pattern P2 extracted by the non-identification element extraction unit 102 in the embodiment. In this example, the non-identification element extraction unit 102 extracts 18 first pattern elements from a second pattern element WE1 to a second pattern element WE20 shown in Fig. 7. Here, Fig. 8 shows an example of an image obtained by superimposing an image of the first pattern element and an image of the second pattern element extracted by the non-identification element extraction unit 102.

Fig. 8 is a diagram showing the example of the image obtained by superimposing the image of the first pattern element and the image of the second pattern element extracted by the non-identification element extraction unit 102 in the embodiment. The image of the first pattern element and the image of the second pattern element may overlap or may not overlap. In the specific example in Fig. 8, the first pattern element SE1 and the second pattern element WE1 overlap. Furthermore, the first pattern element SE20 and the second pattern element WE20 overlap. On the other hand, the second pattern element WE2 and the second pattern element WE4 and the other first pattern elements do not overlap.

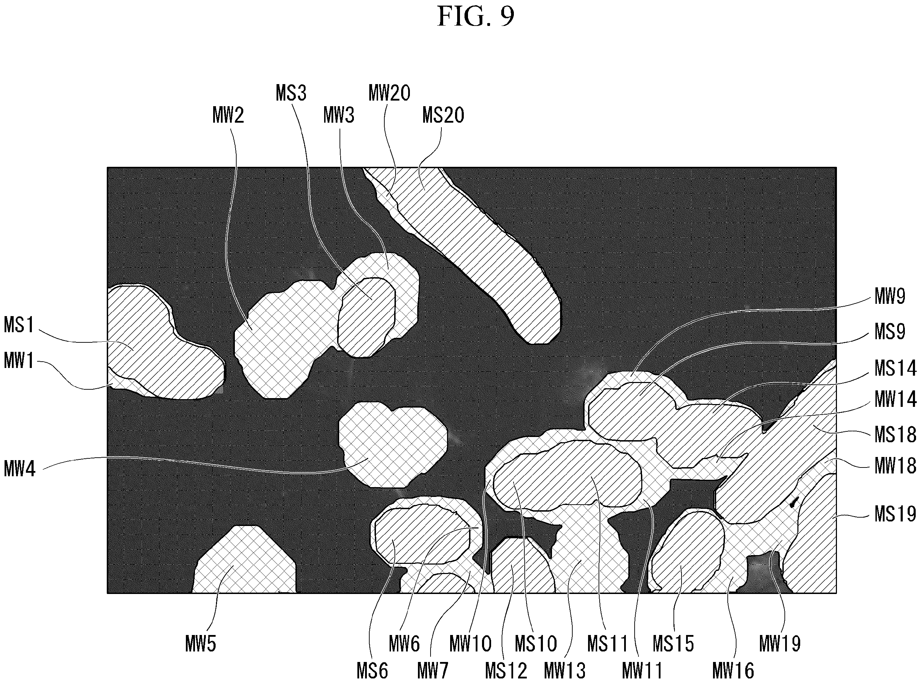

Referring again to Fig. 3, the mask generation unit 103 generates an image mask (Step S40). An example of the image mask generated by the mask generation unit 103 will be described with reference to Fig. 9.

Fig. 9 is a diagram showing the example of the image mask generated by the mask generation unit 103 in the embodiment. The mask generation unit 103 generates a mask corresponding to the image of the non-nucleus element on the basis of the image of the non-nucleus element. In the specific example shown in Fig. 9, the mask generation unit 103 generates a first pattern mask MS1 on the basis of the first pattern element SE1. Furthermore, the mask generation unit 103 generates a second pattern mask MW1 on the basis of the second pattern element WE1. Here, there are various methods for generating an image mask by the mask generation unit 103. In this example, the mask generation unit 103 generates an image mask by performing dilation processing on an image. To be more specific, the mask generation unit 103 dilates an image of the first pattern element SE1 by a predetermined number of pixels. In this example, the predetermined number of pixels is thirty. In other words, in this example, the mask generation unit 103 dilates the image of the first pattern element SE1 by 30 pixels. As a result of this dilation processing, the image of the first pattern element SE1 becomes the first pattern mask MS1. That is to say, the mask generation unit 103 generates the first pattern mask MS 1 on the basis of the image of the first pattern element SE1.

Also, the mask generation unit 103 also generates an image mask for the second pattern element WE1 in the same manner as described above. To be specific, the mask generation unit 103 dilates the image of the second pattern element WE1 by 30 pixels. As a result of this dilation processing, the image of the second pattern element WE1 becomes the second pattern mask MW1. In other words, the mask generation unit 103 generates the second pattern mask MW1 on the basis of the image of the second pattern element WE1. The mask generation unit 103 generates an image mask for the other images with the second pattern P2 and the other images with the first pattern P1 in the same manner as described above. That is to say, the mask generation unit 103 generates a plurality of kinds of image masks according to a status of the image of the non-nucleus element.

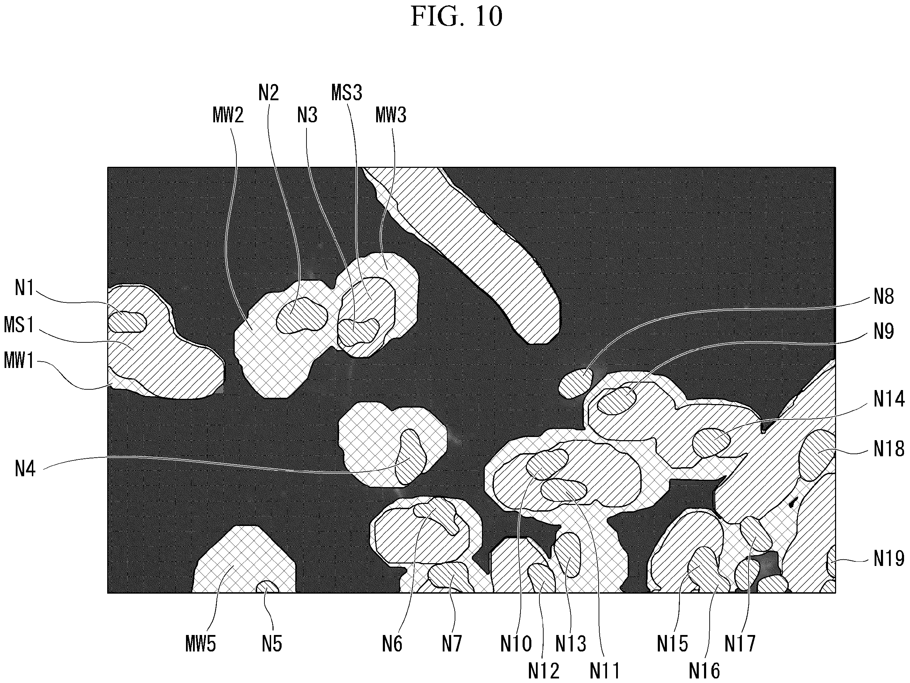

The linking unit 104 links the image of the cell nucleus with the image of the non-nucleus element (Step S50). An example of a linking result by the linking unit 104 will be described with reference to Fig. 10.

Fig. 10 is a diagram showing the example of the linking result by the linking unit 104 in the embodiment. The linking unit 104 acquires the image of the cell nucleus shown in Fig. 4 (the cell nucleus image N1 to the cell nucleus image N19) and the image mask shown in Fig. 9. When the acquired image of the cell nucleus is included in any image mask, the linking unit 104 links the image of the cell nucleus with the image mask. As described above, the image mask is generated on the basis of the image of the non-nucleus element. The linking of the image of the cell nucleus with the image mask is the linking of the image of the cell nucleus with the image of the non-nucleus element.

Here, the image of the cell nucleus is included in any image mask between the first pattern mask MS and the second pattern mask MW in some cases. Furthermore, the image of the cell nucleus is included only in the image mask of the second pattern mask MW in some cases. In addition, the image of the cell nucleus is not included in any image mask in some cases. In the specific example shown in Fig. 10, the cell nucleus image N1 is included in any image mask between the first pattern mask MS1 and the second pattern mask MW1. A cell nucleus image N3 is included in any image mask between the first pattern mask MS3 and the second pattern mask MW3. On the other hand, the cell nucleus image N2 is included only in the image mask of the second pattern mask MW2. Furthermore, a cell nucleus image N8 is not included in any image mask.

The linking unit 104 links the cell nucleus image N1 with the first pattern mask MS1 and the second pattern mask MW1. The linking unit 104 links the cell nucleus image N2 with the second pattern mask MW2. The linking unit 104 does not link any image masks with the cell nucleus image N8. The linking unit 104 links the cell nucleus image N with the image mask with respect to the cell nucleus images N from the cell nucleus image N4 to the cell nucleus image N19 in the same manner as described above.

That is to say, the linking unit 104 links the image of the cell nucleus with the image of the non-nucleus element by linking the image mask generated by the mask generation unit 103 with the image of the cell nucleus.

It should be noted that, although a case in which the linking unit 104 links the cell nucleus with the non-nucleus element in accordance with whether the image of the cell nucleus is included in the image mask has been described in this example, the present invention is not limited thereto. The linking unit 104 may link the image of the cell nucleus with the image of the non-nucleus element further on the basis of a distance between cell nuclei indicated in the image of the cell nuclei. For example, when a distance between images of a plurality of cell nuclei is a predetermined value or less, the images of the plurality of cell nuclei could be included in one image mask in some cases. In such a case, the linking unit 104 may link the images of these plurality of cell nuclei with the one image mask. That is to say, there are a plurality of linking rules of the images of the cell nuclei and the image mask in some cases in accordance with a distance between the cell nuclei indicated by an image of the cell nuclei. In this case, the linking unit 104 may adopt a linking rule according to a distance between the cell nuclei on the basis of the distance.

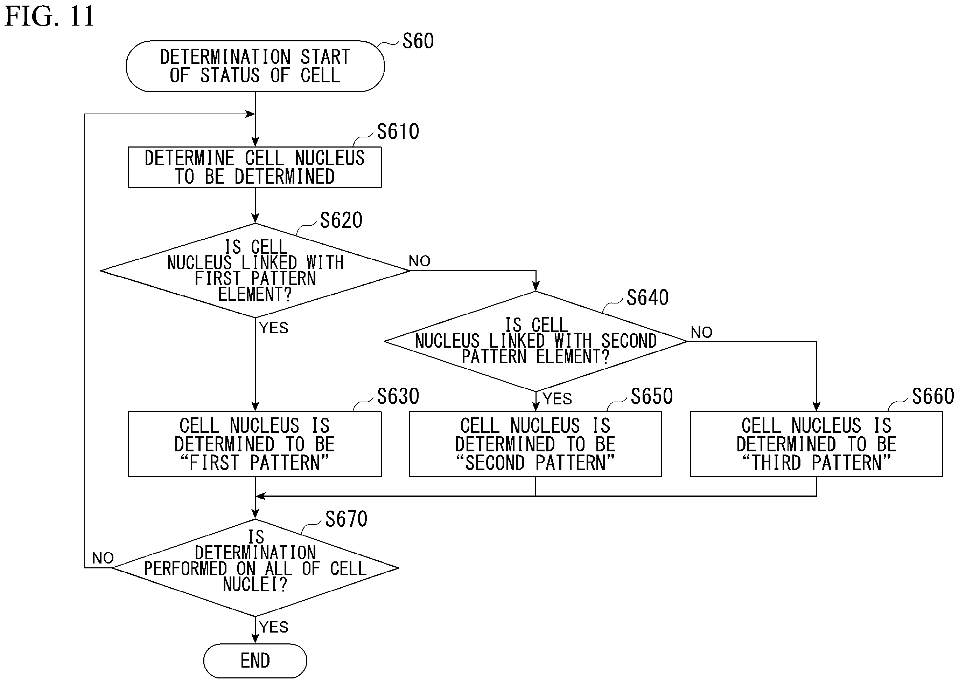

Referring again to Fig. 3, the status determination unit 105 determines a status of the cell on the basis of the image of the cell nucleus and the image of the non-nucleus element linked by the linking unit 104 (Step S60). A detailed specific example of a determination operation for a status of the cell by the status determination unit 105 will be described with reference to Fig. 11.

Fig. 11 is a diagram showing the example of the determination operation by the status determination unit 105 in the embodiment. The status determination unit 105 decides a cell nucleus image N to be determined (Step S610). To be specific, the status determination unit 105 selects the cell nucleus image N to be determined from the plurality of cell nucleus images N from the cell nucleus image N1 to the cell nucleus image N19 shown in Fig. 4. This selection may be performed by a user and, for example, a constitution in which determination target conditions are set in advance and selection is automatically performed may be provided.

The status determination unit 105 determines whether the cell nucleus image N is linked with the first pattern element (Step S620). Here, the linking of the cell nucleus image N with the first pattern element is the linking of the cell nucleus image N with the first pattern mask MS. That is to say, the status determination unit 105 determines whether the cell nucleus image N is linked with the first pattern mask MS. When it is determined that the cell nucleus image N is linked with the first pattern mask MS (Step S620; YES), the status determination unit 105 determines the cell nucleus images N to be the "first pattern P1" (Step S630).

On the other hand, when it is determined that the cell nucleus image N is not linked with the first pattern mask MS (Step S620; NO), the status determination unit 105 determines whether the cell nucleus image N is linked with the second pattern element (Step S640). Here, the linking of the cell nucleus image N with the second pattern element is the linking of the cell nucleus image N with the second pattern mask MW. That is to say, the status determination unit 105 determines whether the cell nucleus image N is linked with the second pattern mask MW. When it is determined that the cell nucleus image N is linked with the second pattern mask MW (Step S640; YES), the status determination unit 105 determines the cell nucleus images N to be the "second pattern P2" (Step S650).

Also, when it is determined that the cell nucleus image N is not linked with the second pattern mask MW (Step S640; NO), the status determination unit 105 determines the cell nucleus images N to be a "third pattern P3" (Step S660). That is to say, the status determination unit 105 determines a status of the cell further on the basis of a kind of an image mask linked with the image of the cell nucleus.

The status determination unit 105 determines whether determination is performed on all of the cell nucleus images N included in the observation image OP (Step S670). In addition, when it is determined that the determination is performed on all of the cell nucleus image N, the series of processes end.

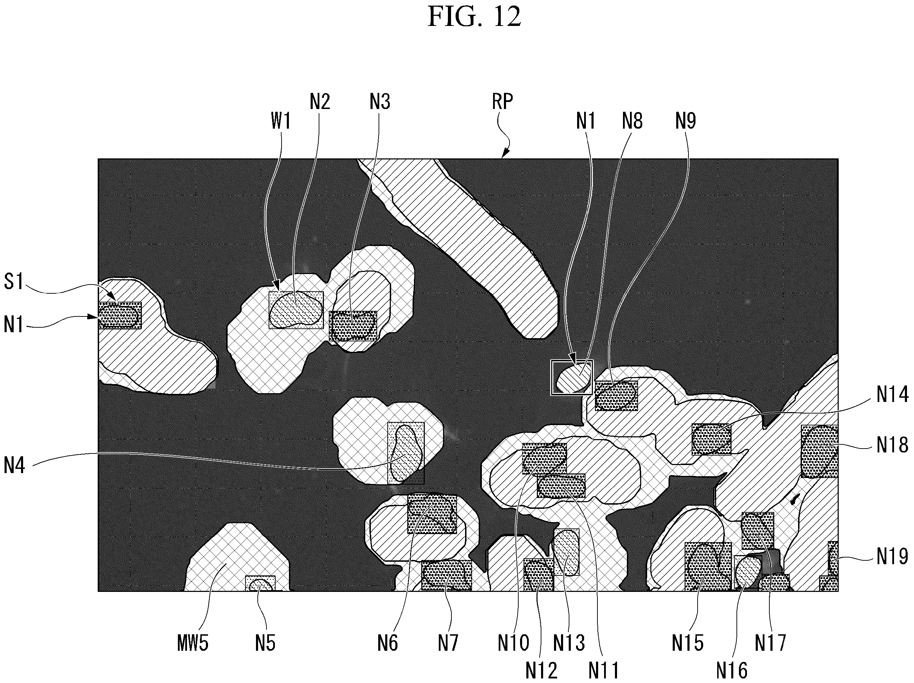

Fig. 12 shows a specific example of a determination result by the status determination unit 105.

Fig. 12 is a diagram showing the example of the determination result by the status determination unit 105 in the embodiment. The status determination unit 105 determines that the cell nucleus image N1 is linked with the first pattern mask MS1. In this case, the status determination unit 105 determines the cell nucleus image N1 to be the "first pattern P1." The status determination unit 105 determines that the cell nucleus image N2 is linked with the second pattern mask MW2. In this case, the status determination unit 105 determines the cell nucleus images N2 to be the "second pattern P2." Furthermore, the status determination unit 105 determines that the cell nucleus image N8 is not linked with any image mask. In this case, the status determination unit 105 determines the cell nucleus image N8 to be the "third pattern P3." That is to say, the status determination unit 105 determines a status of the cell on the basis of the image mask generated by the mask generation unit 103 and the image of the cell nucleus linked with the image mask.

The status determination unit 105 sets the "first pattern P1," the "second pattern P2," and the "third pattern P3" to different images and outputs the images to a result output unit 300. To be specific, the status determination unit 105 generates an image of a first pattern P1 frame SI around the cell nucleus image N1 determined to be the "first pattern P1." The status determination unit 105 generates an image of a second pattern P2 frame WI around the cell nucleus image N2 determined to be the "second pattern P2." Furthermore, the status determination unit 105 generates an image of a third pattern frame NI around the cell nucleus image N8 determined to be the "third pattern P3." The result output unit 300 allows the display unit 30 to display the first pattern P1 frame SI indicating the "first pattern P1," a second pattern P2 frame WI indicating the "second pattern P2," and the third pattern P3 frame NI indicating the "third pattern P3." As a result, the display unit 30 is allowed to display a result image RP shown in Fig. 12.

As described above, the image processing device 10 in the embodiment links the image of the cell nucleus with the image of the non-nucleus element. Thus, it is possible to determine whether a structure for a cell is derived from which cell among a plurality of cells. That is to say, according to the image processing device 10 in the embodiment, the determination concerning whether the structure for the cell is derived from which cell among the plurality of cells can be automatically performed without manual intervention. Thus, it is possible to reduce the labor of evaluating a cell culture status.

Also, the image processing device 10 generates an image mask corresponding to the image of the non-nucleus element on the basis of the image of the non-nucleus element. The image processing device 10 links the generated image mask with the image of the cell nucleus. Here, when the image of the cell nucleus is linked with the image of the non-nucleus element without using an image mask, it is difficult to link the image of the cell nucleus with the image of the non-nucleus element in some cases in accordance with a status of the non-nucleus element. For example, when the non-nucleus element is a dendrite in a neuron, it is easy to link the image of the cell nucleus with an image of the dendrite if the image of the cell nucleus is in contact with the image of the dendrite. However, in the observation image OP, the image of the cell nucleus and the image of the dendrite appear at positions away from each other without in contact with each other. In this case, it is difficult to determine whether the image of the cell nucleus may be linked with any image of the dendrite in some cases. When the image processing device 10 in the embodiment generates an image mask based on the image of the non-nucleus element, a case in which the image of the cell nucleus is included in the image mask occurs in some cases. When the image of the cell nucleus is included in the image mask, the image processing device 10 links the image of the non-nucleus element which is a source of the image mask with the image of the cell nucleus. With such a constitution, the image processing device 10 can easily link the images with each other as compared with a case in which an image mask is not used.

Also, the status determination unit 105 in the image processing device 10 determines a status of the cell on the basis of the image mask generated by the mask generation unit 103 and the image of the cell nucleus linked with the image mask. Here, there are image masks of a plurality of types. In the above-described specific examples, there are image masks of two types, i.e., the image mask with the first pattern P1 and the image mask with the second pattern P2. That is to say, the status determination unit 105 determines a status of the cell on the basis of a correspondence between a type of image mask and an image of a cell nucleus included in this image mask. Here, when the status of the cell is attempted to be determined by comparing the image of the cell nucleus with the image of the non-nucleus element without using an image mask, the labor of calculating the feature quantities of the images and then comparing the feature quantities is required. In other words, when the status of the cell is attempted to be determined without using an image mask, the computation for determination is complicated. The status determination unit 105 in the embodiment determines the status of the cell on the basis of the type of image mask. Thus, the status of the cell can be determined without complicating the computation for determination.

In the determination of the status of the cell, the status of the cell can be determined by distinguishing, as a cell, a region including a cell nucleus which is an identification element from a cell and then determining whether a region which is a non-identification element and is for determining a differentiation status of a cell is present around the nucleus. When such differentiation of the cell progresses, it is possible to determine a differentiation status of the cell by linking (associating) whether an existing region is present around the cell nucleus. When the differentiation of the cell progresses around the cell nucleus, if there is no existing region around the cell nucleus, it can be determined that the differentiation of the cell does not progress. In this case, there is a region which is not the region in which the differentiation of the cell present around the cell nucleus progresses and it is possible to determine a status of the cell by linking (associating) this region with the cell nucleus.

Also, the linking unit 104 in the image processing device 10 links the image of the cell nucleus with the image of the non-nucleus element further on the basis of the distance between the cell nuclei indicated by the image of the cell nuclei. Here, if dilation processing is performed on the image of the non-nucleus element when the image mask is generated, a plurality of images of the cell nucleus are included in the image mask in some cases. Even in such a case, the linking unit 104 can link the images while performing determination concerning whether the image of the cell nucleus is linked with which image mask on the basis of the distance between the cell nuclei. That is to say, according to the image processing device 10 including the linking unit 104, it is possible to link the image of the cell nucleus with the image of the non-nucleus element with high accuracy.

It should be noted that, although a case in which the identification element extraction unit 101 extracts the image of the cell nucleus and the non-identification element extraction unit 102 extracts the element which is not the cell nucleus, that is, the non-nucleus element, has been described in the above description, the present invention is not limited thereto.

It should be noted that a program for executing each processing of the observation device 1 or the image processing device 10 in the embodiments of the present invention is recorded on a computer readable recording medium and the above-described various processes may be performed by reading and executing the program recorded on the recording medium.

It should be noted that a "computer system" mentioned herein may include an operating system (OS) and hardware such as peripheral devices. Furthermore, the "computer system" is a system which also includes a home page provision environment (or display environment) when the computer system uses a world wide web (WWW) system. In addition, a "computer readable recording medium" refers to a storage device such as a non-volatile memory such as a flexible disk, a magneto-optical disk, a read only memory (ROM), and a flash memory, a portable medium such as a compact disc (CD)-ROM, and a hard disk built in a computer system.

Moreover, a "computer readable recording medium" also includes a medium which holds a program for a certain period of time like a volatile memory (for example, a dynamic random access memory (DRAM)) inside a computer system serving as a server or a client when a program is transmitted via a network such as the Internet or a communication circuit such as a telephone circuit. Furthermore, the program may be transmitted from a computer system having a storage device or the like which stores the program to another computer system via a transmission medium or through a transmission wave in a transmission medium. Here, a "transmission medium" which transmits a program refers to a medium which has a function of transmitting information like a network (communication network) such as the Internet or a communication circuit (communication line) such as a telephone circuit. The program may be a program configured to realize some of the above-described functions. The program may be a differential file (differential program) which can be realized by combining the above-described functions with a program recorded in the computer system in advance.

While the embodiments of the present invention have been described in detail above with reference to the drawings, the specific constitutions are not limited to the embodiments and also include designs within a range which does not deviate from the gist of the present invention.

### [Reference Signs List]

1 observation device
10 image processing device
20 microscope device
22 imaging unit
30 display unit
100 calculation unit
101 identification element extraction unit
102 non-identification element extraction unit
103 mask generation unit
104 linking unit
105 status determination unit
200 storage unit
300 result output unit

## Claims

1. An image processing device comprising:
an associating unit configured to associate information on an identification element used to identify a cell in imaged cell information with information on a non-identification element which is an element of the cell which is not the identification element on the basis of the information on the identification element and the information of the non-identification element; and
a status determination unit configured to determine a status of the cell on the basis of the information on the identification element and the information on the non-identification element associated together by the linking unit.

2. The image processing device according to claim 1, further comprising:
a mask generation unit configured to generate an image mask corresponding to the information on the non-identification element on the basis of the information on the non-identification element,
wherein the linking unit associates the information on the identification element with the information on the non-identification element by associating the image mask generated by the mask generation unit with the information on the identification element.

3. The image processing device according to claim 2, wherein the status determination unit determines a status of the cell on the basis of the image mask generated by the mask generation unit and the information on the identification element associated with the image mask.

4. The image processing device according to claim 2 or 3, wherein the mask generation unit generates image masks of a plurality of types according to a status of the information on the non-identification element and
the status determination unit determines a status of the cell further on the basis of a type of the image mask associated with the information on the identification element.

5. The image processing device according to any one of claims 1 to 4, wherein the linking unit associates the information on the identification element with the information on the non-identification element further on the basis of a distance between identification elements indicated by the information on the identification element.

6. The image processing device according to any one of claims 1 to 5, wherein the information is a cell image obtained by capturing an image of cells.

7. The image processing device according to any one of claims 1 to 6, wherein the information on the non-identification element includes pieces of information indicating a plurality of different statuses of the cell in accordance with the acquired information.

8. The image processing device according to claim 7, wherein the determination device determines a status of the cell in accordance with the result of association between each piece of information of the plurality of pieces of different information and the information on the identification element.

9. The image processing device according to any one of claims 1 to 8, wherein the information indicating the plurality of different statuses of the cell in the non-identification element includes pieces of information indicating different statuses of the cell for determining the status of the cell and the determination device determines a status in accordance with whether any of the pieces of information indicating the different statuses of the cell is associated with the information on the identification element.

10. An observation device comprising:
an imaging unit configured to output an image having a cell captured therein; and
the image processing device according to any one of claims 1 to 9.

11. A program which causes a computer to execute:
an association step of associating information on an identification element used to identify a cell in a captured cell image with information on a non-identification element serving as an element of the cell which is not the identification element on the basis of the identification element and the information on the non-identification element; and
a status determination step of determining a status of the cell on the basis of the information on the identification element and the information on the non-identification element associated with each other in the association step.
